# EUROPEAN PATENT SPECIFICATION

(11) **EP 2 689 791 B1**
(45) Date of publication and mention of the grant of the patent: **22.04.2015**
(21) Application number: 13178130.4
(22) Date of filing: 26.07.2013
(51) Int. Cl.: A61M 1/16

(54) **Extracorporeal circuit for removing co2 from blood**
Extrakorporaler kreislauf zur entfernung von co2 aus blut
Circuit extracorporel pour enlever du co2 du sang

(30) Priority: 26.07.2012 IT BO20120404
(43) Date of publication of application: 29.01.2014
(73) Proprietor: Patroniti, Nicolò Antonino, Lissone (IT); Pesenti, Antonio, Milano (IT)
(72) Inventor: Patroniti, Nicolò Antonino, Lissone (IT); Pesenti, Antonio, Milano (IT)
(74) Representative: Bosman, Cesare

(56) References cited:
- WO-A2-2008/135282
- DE-C1- 4 028 311
- US-A1- 2012 035 523

## Description

The present invention concerns an extracorporeal circuit for removing CO₂ from blood.

In the past, various solutions have been devised for removing CO₂ from blood. Many of these solutions entail extracorporeal treatment of the blood, analogously to the process of dialysis which removes toxic or harmful substances which should normally be eliminated by the kidneys.

Removal of CO₂ from blood is necessary when the function of the lungs is damaged due to various pathologies and has to be substituted, even only partially.

In general, extracorporeal circuits for removing CO₂ from blood entail the venous blood being excluded from the lung circuit in order for it to be artificially oxygenated by means of a gas exchanger, such as an oxygenator. More specifically, the venous blood which returns towards the right atrium of the heart is collected in the extracorporeal circuit and pumped into the oxygenator from which it is then conveyed into the arterial circuit, thus by-passing the heart and the pulmonary circulation.

The most widely used oxygenation devices are membrane oxygenators in which the blood and the oxygen (or an oxygen-rich mixture) flow from opposite parts of a membrane. These types of devices therefore act on the quantity of CO₂ dissolved in the blood which, in reality, is a limited portion of the total CO₂ content. In fact, it is known that 90% of the CO₂ transported in the blood is in the form of carbonate ions according to the following chemical equilibrium (I).

CO₂ + H₂0 ↔ H₂CO₃ ↔ H⁺ + HCO₃⁻ (I)

One solution in order to increase the removal of CO₂ from blood by means of membrane oxygenators could be to intervene on the chemical equilibrium (I), inducing an increase in the percentage of gaseous CO₂. In this way, the increase in concentration of gaseous CO₂ to be removed by the oxygenator promotes removal of the total CO₂.

One way of intervening on the chemical equilibrium (I) in favour of the gaseous CO₂ is acidification from the outside. Said acidification is achieved by the addition of acid substance in the extracorporeal circuit upstream of the oxygenator.

As may seem obvious to a person skilled in the art, said addition of acid can entail a series of drawbacks which must be resolved in order not to endanger the health of the patient.

The object of the present invention is to produce an extracorporeal circuit for removing CO₂ from blood, able to carry out the above-mentioned acidification which promotes shifting of the equilibrium (I) in favour of the gaseous CO₂ and at the same time guarantees high safety levels.

The subject of the present invention is an extracorporeal circuit for removing CO₂ from blood, the essential characteristics of which are reported in claim 1, and the preferred and/or auxiliary characteristics of which are reported in claims 2-5.

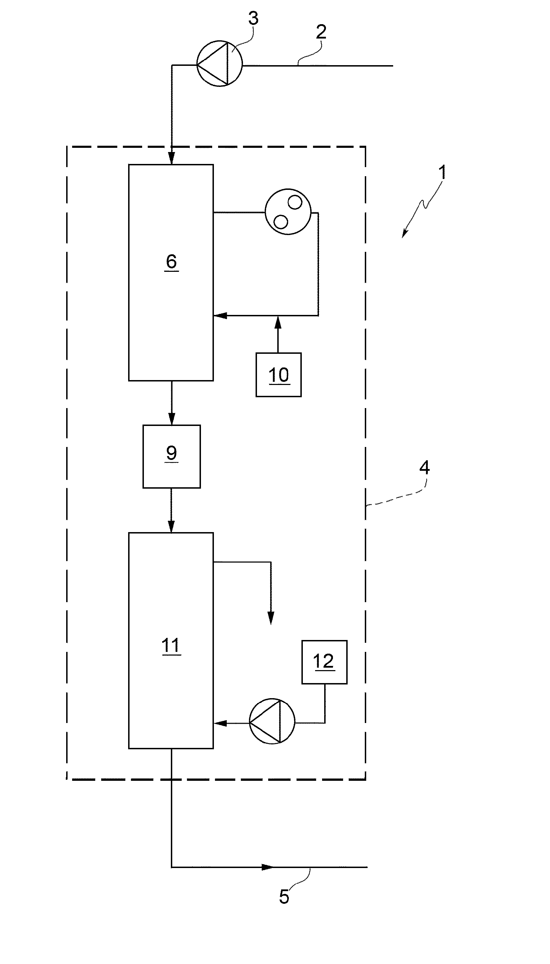

For a better understanding of the invention, an embodiment is provided below, for purely illustrative non-limiting purposes with the help of the accompanying figure, which schematically illustrates an extracorporeal circuit for removing blood according to the present invention.

In the figure, the number 1 indicates as a whole an extracorporeal circuit for removing blood subject of the present invention.

The circuit 1 comprises a blood taking line 2 for taking blood from the patient, on which a peristaltic pump 3 operates, an oxygenation assembly 4 and a blood re-introduction line 5 for re-introducing the blood into the patient operate.

The oxygenation assembly 4 comprises a dialyzer 6 connected to a circulation circuit 7 for the circulation of a dialyzer bath operated by a peristaltic pump 8 and an oxygenator 9 arranged downstream of the dialyzer 6 following the flow of the blood in the extracorporeal circuit 1. In particular, the dialyzer 6 is a haemofilter and the dialyzer bath is plasma water.

Furthermore, the oxygenator 9 is of the membrane type in which the gases move from a compartment with higher partial pressure to a compartment with lower partial pressure. In the case of the gaseous CO₂, it moves from the blood compartment to the ventilation fluid compartment following the partial pressure gradient.

The oxygenation assembly 4 comprises an acidifier 10 suitable for introducing into the circulation circuit 7 of the dialyzer bath, an acid substance to cause shifting of the chemical equilibrium (I) in favour of the gaseous CO₂. In particular, the acid substance is a mixture of an inorganic acid, such as hydrochloric acid for example, and organic acids, such as pyruvic acid, citric acid and lactic acid already normally present in the organism.

In this way, an increase is induced in the gaseous CO₂ in the plasma water and consequently in the blood which undergoes the action of the oxygenator 9. The increase in partial pressure of the gaseous CO₂ will certainly favour its removal in quantity terms.

The oxygenation assembly 4 furthermore comprises a dialyzer 11 arranged downstream of the oxygenator 9 from which it receives the blood. The dialyzer 11 entails the use of a basic dialyzing solution 12 such as to re-balance the pH modified by acidification of the plasma water in the circulation circuit of the dialyzer bath.

It has been proved that performing the acidification on the plasma water instead of directly on the blood guarantees a greater efficiency both in terms of effectiveness of the acidification itself and in terms of safety.

## Claims

1. An extracorporeal circuit (1) for removing CO₂ from blood comprising a blood taking line (2) for taking blood from the patient, an oxygenation assembly (4), and a blood re-introduction line (5) for re-introducing blood into the patient; said extracorporeal circuit being **characterised in that** said oxygenation assembly (4) comprises an oxygenator (9), a first dialyzer (6), which is arranged upstream from the oxygenator (9), a circulation circuit (7) for the circulation of a dialyzer bath and connected to the dialyzer (6), an acidifier (10), which is suited to introduce an acid substance into the circulation circuit (7) of the dialyzer bath, and basic neutralization means (11, 12) which are arranged downstream of oxygenator (9).

2. The extracorporeal circuit (1) for removing CO₂ from blood according to claim 1, **characterised in that** the basic neutralization means comprise a second dialyzer (11), in which a respective basic dialyzing solution (12) circulates.

3. The extracorporeal circuit (1) for removing CO₂ from blood according to claim 1 or 2, **characterised in that** said circulation circuit (7) of the dialyzer bath is a circulation circuit of plasma water.

4. The extracorporeal circuit (1) for removing CO₂ from blood according to claim 3, **characterised in that** said first dialyzer (6) is a haemofilter.

5. The extracorporeal circuit (1) for removing CO₂ from blood according to any of the previous claims, **characterised in that** said oxygenator (9) is a membrane oxygenator.

## Patentansprüche

1. Extrakorporaler Kreislauf (1) zum Entfernen von CO₂ aus Blut, umfassend eine Blutentnahmeleitung (2) zur Entnahme von Blut von dem Patienten, eine Oxygenierungseinrichtung (4) und eine Blutrückführungsleitung (5) zum Rückführen von Blut in den Patienten; wobei der extrakorporale Kreislauf **dadurch gekennzeichnet ist, dass** die Oxygenierungseinrichtung (4) einen Oxygenator (9), einen ersten Dialysator (6), der dem Oxygenator (9) vorgeschaltet ist, einen mit dem Dialysator (6) verbunden Zirkulationskreislauf (7) zur Zirkulation eines Dialysatorbads, ein Mittel zum Ansäuern (10), das zum Einbringen einer sauren Substanz in den Zirkulationskreislauf (7) des Dialysatorbads geeignet ist, und basische Neutralisierungsmittel (11, 12), die Oxygenator (9) nachgeschaltet sind, umfasst.

2. Extrakorporaler Kreislauf (1) zum Entfernen von CO₂ aus Blut nach Anspruch 1, **dadurch gekennzeichnet, dass** die basischen Neutralisierungsmittel einen zweiten Dialysator (11) umfassen, in dem eine entsprechende basische Dialyselösung (12) zirkuliert.

3. Extrakorporaler Kreislauf (1) zum Entfernen von CO₂ aus Blut nach Anspruch 1 oder 2, **dadurch gekennzeichnet, dass** der Zirkulationskreislauf (7) des Dialysatorbads ein Zirkulationskreislauf für Plasmawasser ist.

4. Extrakorporaler Kreislauf (1) zum Entfernen von CO₂ aus Blut nach Anspruch 3, **dadurch gekennzeichnet, dass** der erste Dialysator (6) ein Hämofilter ist.

5. Extrakorporaler Kreislauf (1) zum Entfernen von CO₂ aus Blut nach einem der vorhergehenden Ansprüche, **dadurch gekennzeichnet, dass** der Oxygenator (9) ein Membranoxygenator ist.

## Revendications

1. Circuit extracorporel (1) pour l'élimination de CO2 du sang comprenant une ligne de prélèvement de sang (2) pour le prélèvement du sang du patient, un ensemble d'oxygénation (4), et une ligne de réintroduction de sang (5) pour la réintroduction du sang chez le patient ; ledit circuit extracorporel étant **caractérisé en ce que** ledit ensemble d'oxygénation (4) comprend un oxygénateur (9), un premier dialyseur (6), qui est agencé en amont de l'oxygénateur (9), un circuit de circulation (7), pour la circulation d'un bain de dialyseur et raccordé au dialyseur (6), un dispositif d'acidification (10), qui est adapté pour introduire une substance acide dans le circuit de circulation (7) du bain de dialyseur, et des moyens de neutralisation basique (11, 12) qui sont agencés en aval de l'oxygénateur (9).

2. Circuit extracorporel (1) pour l'élimination de CO2 du de sang selon la revendication 1, **caractérisé en ce que** les moyens de neutralisation basique comprennent un deuxième dialyseur (11), dans lequel une solution de dialyse basique respective (12) circule.

3. Circuit extracorporel (1) pour l'élimination de CO2 du sang selon la revendication 1 ou 2, **caractérisé en ce que** ledit circuit de circulation (7) du bain de dialyseur est un circuit de circulation d'eau plasmatique.

4. Circuit extracorporel (1) pour l'élimination de CO2 du sang selon la revendication 3, **caractérisé en ce que** ledit premier dialyseur (6) est un hémofiltre.

5. Circuit extracorporel (1) pour l'élimination de CO2 du sang selon l'une des revendications précédentes, **caractérisé en ce que** ledit oxygénateur (9) est un oxygénateur à membrane.
